# EUROPEAN PATENT SPECIFICATION

(11) **EP 1 595 959 B1**
(45) Date of publication and mention of the grant of the patent: **21.01.2015**
(21) Application number: 04712210.6
(22) Date of filing: 18.02.2004
(51) Int. Cl.: C12Q 1/68, A61K 39/395, A61P 31/18, C12N 15/49, C07K 16/10

(54) **METHOD FOR ENHANCING EFFICACY OF A MONOCLONAL ANTIBODY PREPARATION**
VERFAHREN ZUR VERSTÄRKUNG DER WIRKSAMKEIT EINES PRÄPARATS VON MONOKLONALEM ANTIKÖRPER
PROCEDE VISANT A AUGMENTER L'EFFICACITE D'UNE PREPARATION D'UN ANTICORPS MONOCLONAL

(30) Priority: 20.02.2003 JP 2003042819
(43) Date of publication of application: 16.11.2005
(73) Proprietor: The Chemo-Sero-Therapeutic Research Institute, Kumamoto-shi Kumamoto 860-8568 (JP)
(72) Inventor: MURAKAMI, Toshio, Kikuchi-shi, Kumamoto 869-1298 (JP); HIGUCHI, Hirofumi, Kikuchi-shi, Kumamoto 869-1298 (JP); MAKIZUMI, Keiichi, Kikuchi-shi, Kumamoto 869-1298 (JP); MAEDA, Toshihiro, Kikuchi-shi, Kumamoto 869-1298 (JP); MIZOKAMI, Hiroshi, Kikuchi-shi, Kumamoto 869-1298 (JP)
(74) Representative: Vossius & Partner
(86) International application number: PCT/JP2004/001822
(87) International publication number: WO 2004/074516

(56) References cited:
- EP-A- 0 690 132
- WO-A-90/15078
- US-A- 5 712 373
- US-A- 5 858 369
- US-B1- 6 447 778
- WANG FENG-XIANG ET AL: "Emergence of autologous neutralization-resistant variants from preexisting human immunodeficiency virus (HIV) quasi species during virus rebound in HIV type 1-infected patients undergoing highly active antiretroviral therapy." THE JOURNAL OF INFECTIOUS DISEASES. 1 MAR 2002, vol. 185, no. 5, 1 March 2002 (2002-03-01), pages 608-617, XP002377523 ISSN: 0022-1899
- ZVI A ET AL: "The principal neutralizing determinant of HIV-1 located in V3 of gp120 forms a 12-residue loop by internal hydrophobic interactions." FEBS LETTERS. 17 JUL 1995, vol. 368, no. 2, 17 July 1995 (1995-07-17), pages 267-270, XP002377524 ISSN: 0014-5793
- TUGARINOV V ET AL: "NMR structure of an anti-gp120 antibody complex with a V3 peptide reveals a surface important for co-receptor binding." STRUCTURE WITH FOLDING & DESIGN. 15 APR 2000, vol. 8, no. 4, 15 April 2000 (2000-04-15), pages 385-395, XP002377525 ISSN: 0969-2126
- SHIBATA J, YOSIHMURA K, HONDA A, MURAKAMI T, KOITO A, MATSUSHITA S: "A Role of Mutations in Non-V3 Envelope Regions for Escape from a Broad Neutralizing Anti-V3 Monoclonal Antibody, KD-247, during in vitro Selection" 13TH CONFERENCE ON RETROVIRUSES AND OPPORTUNISTIC INFECTIONS, [Online] 8 February 2006 (2006-02-08), XP002377220 Denver, Colorado Retrieved from the Internet: URL:http://www.retroconference.org/2006/Ab stracts/26741.htm> [retrieved on 2006-02-08]

### SEQUENCE LISTING

<110> JURIDICAL FOUNDATION THE CHEMO-SERO-THERAPEUTIC RESEARCH INSTITUTE
<120> Method for Improving Effectiveness in Prescribing Monoclonal
   Antibody Medicaments for Patients
<130> 663728
<150> JP 2003-42819
   <151> 2003-02-20
<160> 11
<210> 1
   <211> 20
   <212> DNA
   <213> Artificial Sequence
<220>
   <223> Primer
<400> 1
   acacatggaa ttaggccagt 20
<210> 2
   <211> 20
   <212> DNA
   <213> Artificial Sequence
<220>
   <223> Primer
<400> 2
   aaattcccct ccacaattaa 20
<210> 3
   <211> 30
   <212> DNA
   <213> Artificial Sequence
<220>
   <223> Primer
<400> 3
   gccggatcct caactcaact gctgttaaat 30
<210> 4
   <211> 35
   <212> DNA
   <213> Artificial Sequence
<220>
   <223> Primer
<400> 4
   gctctgcagt caaatttctg ggtcccctcc tgagg 35
<210> 5
   <211> 7
   <212> PRT
   <213> HIV-1
<400> 5
   Ile Gly Pro Ala Arg Ala Phe
   1 5
<210> 6
   <211>7
   <212> PRT
   <213> HIV-1
<400> 6
   Ile Gly Pro Gly Arg Ser Phe
   1 5
<210> 7
   <211> 7
   <212> PRT
   <213> HIV-1
<400> 7
   Ile Gly Pro Gly Arg Ala Leu
   1 5
<210> 8
   <211> 7
   <212> PRT
   <213> HIV-1
<400> 8
   Ile Gly Pro Gly Arg Thr Phe
   1 5
<210> 9
   <211> 7
   <212> PRT
   <213> HIV-1
<400> 9
   Ile Gly Pro Gly Arg Ala Ile
   1 5
<210> 10
   <211> 7
   <212> PRT
   <213> HIV-1
<400> 10
   Val Gly Pro Gly Arg Ala Leu
   1 5
<210> 11
   <211> 7
   <212> PRT
   <213> HIV-1
<400> 11
   Ile Gly Pro Gly Arg Ala Phe
   1 5

## Description

### TECHNICAL FIELD

The present invention belongs to the technical field of medicaments. More specifically, the present invention relates to a method for enhancing efficacy of a preparation of a monoclonal antibody as characterized in the appended claims, said method being carried out for selecting patients who possess an antigen recognized by said monoclonal antibody.

### BACKGROUND ART

In recent years, for clinical application of a monoclonal antibody, a therapy with administration of a monoclonal antibody preparation has been developed. A monoclonal antibody preparation may effectively be used through interaction between a monoclonal antibody and an antigen occurring within the living body. Thus, efficacy of a monoclonal antibody preparation may be enhanced if an expression level of said antigen is previously tested to select subjects to be applied with said preparation. A monoclonal antibody may be advantageous from the viewpoint of safety and efficacy due to its extremely high specificity. However, in case that diversity occurs in an amino acid sequence of an antigenic epitope region, a monoclonal antibody will become less reactive with an antigen and hence its effectiveness may be deteriorated. Therefore, for use of a monoclonal antibody preparation as a medicament, it will be a useful means for enhancing efficacy of said monoclonal antibody preparation not only to test previously an expression level of an antigen within the living body but also to confirm diversity of an antigen and to select subjects for administration.

Among monoclonal antibody preparations wherein subjects to be administered therewith are tested and selected is anti-HER2 monoclonal antibody (Trastuzumab) now commercially available. Trastuzumab is a monoclonal antibody to HER2 protein that is overexpressed in about 20 to 30% of breast cancer patients. For clinical test for selecting subjects to be administered with anti-HER2 monoclonal antibody, Southern blot or Furuorescent in situ hybridization (FISH) technique for DNA amplification of HER2/neu gene; Northern blot or reverse transcription polymerase chain reaction (RT-PCR) technique for screening overexpression of HER2 mRNA; or Western blot, ELISA or immunohistochemistry technique for screening overexpression of HER2 protein have been attempted. With these tests, efficacy of Trastuzumab has been enhanced in cases of HER2 overexpression, suggesting that in therapy with a monoclonal antibody preparation selecting previously subjects to be administered with said preparation is useful for enhancing efficacy of the monoclonal antibody preparation (e.g. Nippon Rinsho, Vol. 60, No. 3 (2002)).

### DISCLOSURE OF THE INVENTION

### (Technical Problems to be Solved by the Invention)

On the other hand, in case that there occurs diversity in an amino acid sequence of an epitope region to thereby alter affinity of antigen-antibody reaction, efficacy of an antibody preparation is estimated to be lowered as a consequence of antigenic diversity, prediction of which is of great significance from clinical point of view. For this purpose, testing an expression level of an antigenic protein alone as in Trastuzumab is insufficient and a newly established test method will be necessary. This is because, even if a certain antigen is highly expressed as a protein and is detectable quantitatively, said antigen must be assessed as being unfit if it exhibits diversity in an amino acid sequence of an epitope in wild type recognized by a monoclonal antibody and there are multiplicity of antigenic variants with lowered reactivity with said monoclonal antibody. Accordingly, when there exists diversity in an amino acid sequence of an epitope of a certain antigen, an extent of reactivity of an antibody to each of epitopes with different amino acid sequences needs be directly confirmed through antigen-antibody reaction. However, in order to distinguish antigenic molecules with different amino acid sequences of an epitope region from each other among antigens that may occur in extremely small quantities and to capture and detect said molecules with an antibody, a higher sensitivity will be required. It is also difficult to distinguishably detect an expression level of an antigen on one hand and reactivity of an antibody on the other hand.

From the viewpoints mentioned above, for a monoclonal antibody preparation wherein an antigen to which a monoclonal antibody is directed is a protein with diversity in an amino acid sequence of an epitope recognized by said monoclonal antibody, development of a test method with high sensitivity and rapidity is desired for selection of subjects to be administered with said preparation.

### (Means to Solve the Problems)

The present invention relates to a method for enhancing efficacy of a monoclonal antibody preparation as characterized in the appended claims.

In accordance with the present invention said method comprises steps: (1) deducing an amino acid sequence of a protein expressed in patients from a nucleotide sequence of a gene of a target molecule determined by isolation and analysis of said gene in biopsy from patients; (2) assessing fitness of patients for administration of said monoclonal antibody preparation by comparing the amino acid sequence deduced in step (1) with an amino acid sequence that is recognizable by said monoclonal antibody as previously determined (hereinafter referred to as "reference sequence"); and (3) selecting patients to be administered with said monoclonal antibody preparation with its expected efficacy based on the fitness assessed in step (2).

Diversity in an amino acid sequence of an antigenic epitope recognized by a monoclonal antibody preparation may alter binding affinity of the antibody to the antigen. In case of an antigen that exhibits a genetic polymorphism in an epitope region, the present inventors had the view that, in place of a direct measurement of binding of a monoclonal antibody through immunochemical detection, diversity of an antigen expressed in patients may be analyzed based on analysis of its nucleotide sequence to thereby predict binding between said antigen and said antibody. Thus, for a monoclonal antibody in which correlation was found between an amino acid sequence of an epitope and reactivity of antigen/antibody, data of such correlation were collected. The data thus obtained was then compared to a nucleotide sequence of an epitope region of an antigen from patients to allow for efficient selection of patients to be administered with a monoclonal antibody preparation to complete a method in accordance with the present invention.

With the progress of PCR and nucleotide sequence analysis techniques, the method in accordance with the present invention may be performed with a high sensitivity in a wide use and allows for direct analysis without cloning of DNAs among which their variants are present. Therefore, in accordance with the present invention, prediction of efficacy of a monoclonal antibody preparation now becomes possible by merely determining a nucleotide sequence encoding an epitope region of an antigen of an expressed protein without need of actually measuring binding affinity between said antigen and said monoclonal antibody.

### (More Efficacious Effects than Prior Art)

In accordance with the present invention, a method for enhancing overall efficacy of a monoclonal antibody preparation as characterized in the appended claims is provided, without actually measuring binding affinity between an antigen of an expressed target protein and said monoclonal antibody through isolation and purification of said antigen, by selecting patients to be administered with said monoclonal antibody preparation by previously analyzing a nucleotide sequence that encodes an epitope region of said antigen and by comparing said sequence with an amino acid sequence that is recognizable by said monoclonal antibody as previously determined ("reference sequence") to thereby assess fitness of patients for administration of said monoclonal antibody preparation.

### BRIEF DESCRIPTION OF DRAWINGS

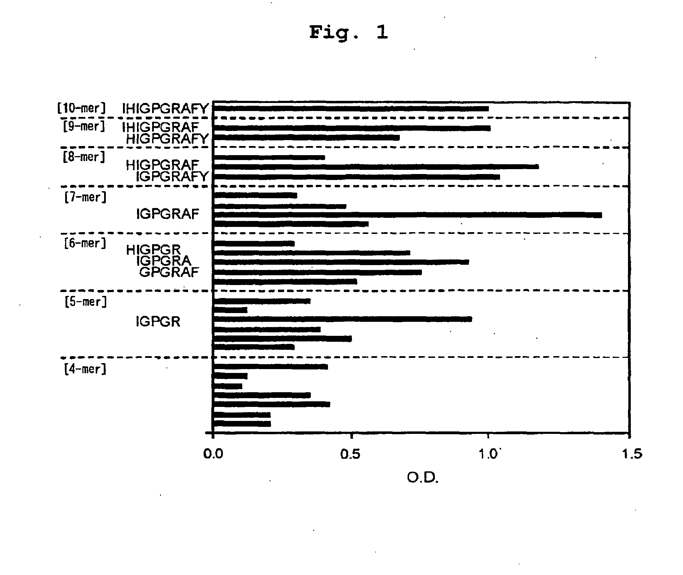

Fig. 1 is a graph showing binding of KD-247 and each of synthetic peptides wherein amino acid residues are sequentially deleted one by one from the synthetic peptide "IHIGPGRAFY", which is the PND region of the laboratory strain of HIV-1 (MN strain) proved to be neutralized by KD-247, for investigation of a site recognized by the monoclonal antibody.

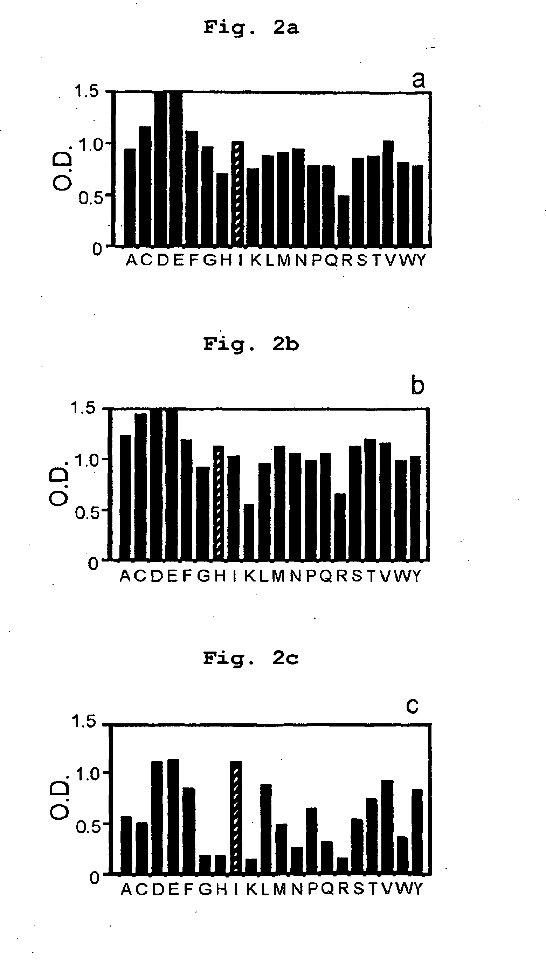

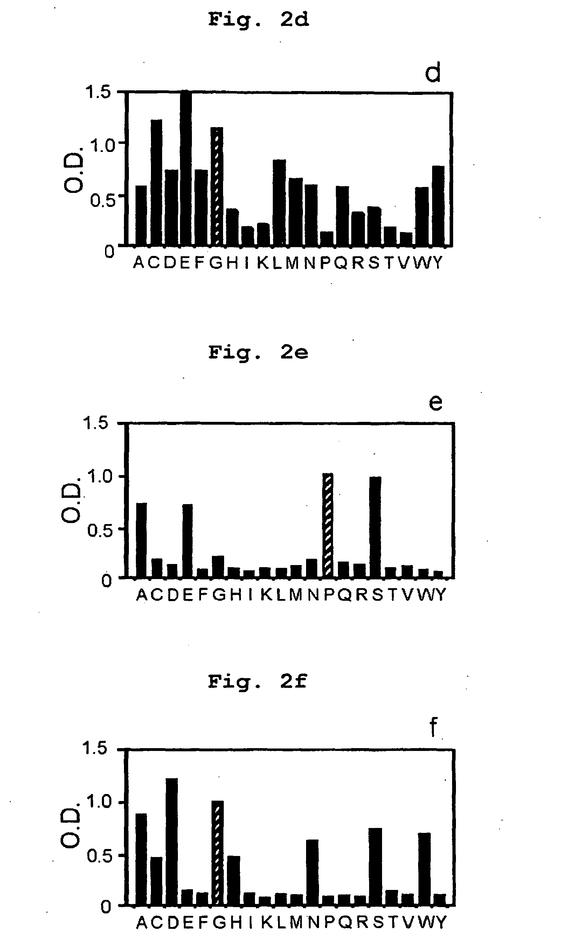

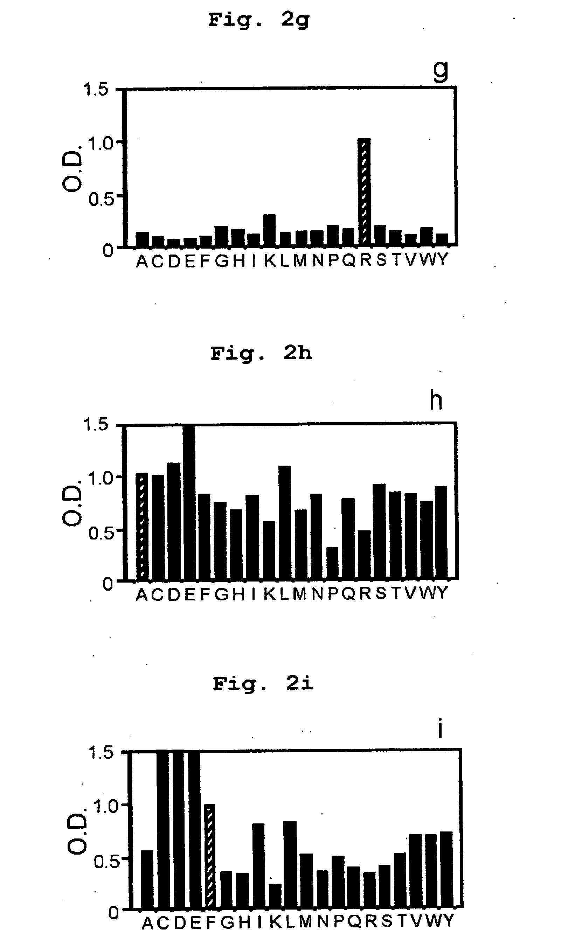

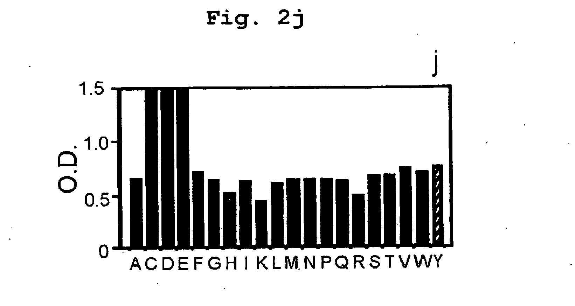

Fig. 2 is a graph showing binding of KD-247 and each of synthetic peptides wherein each of the amino acid residues from the N-terminal "I" to the C-terminal "Y" in the synthetic peptide "IHIGPGRAFY", which is the PND region of the laboratory strain of HIV-1 (MN strain) proved to be neutralized by KD-247, was replaced with the other 19 naturally occurring amino acid residues as shown in Fig. 2a to Fig. 2j, respectively, for investigation of versatility of the amino acid sequence recognized by the monoclonal antibody. In Fig. 2a to Fig. 2j, the bar with a slanting line indicates no alteration of the original amino acid residue.

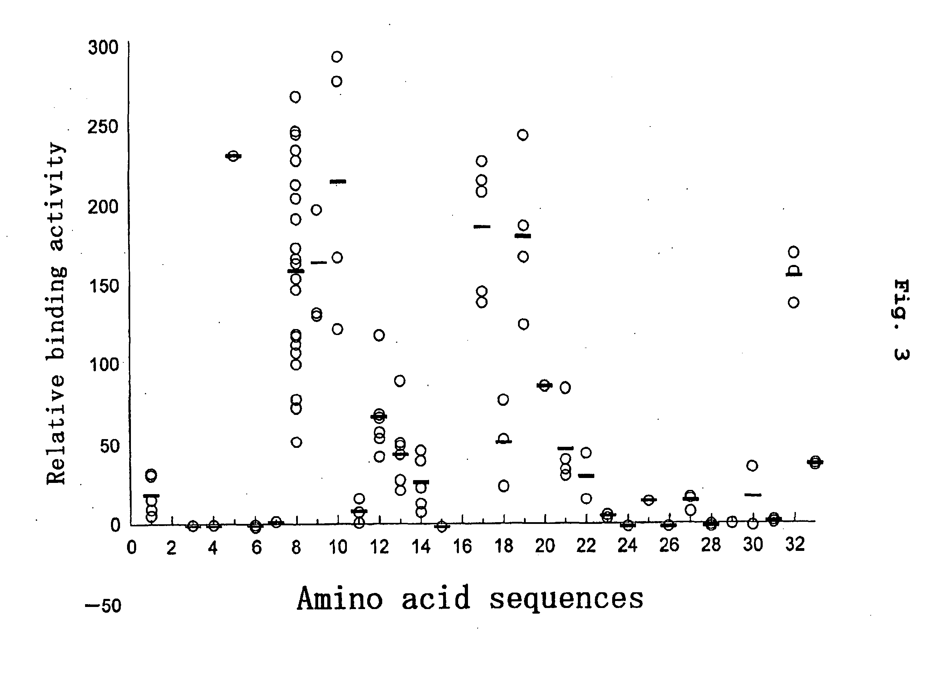

Fig. 3 is a graph showing binding of KD-247 to an amino acid sequence at the middle portion of V3 region of a protein expressed from an amplified HIV-1 gene V3 region from patients, for investigation of the amino acid sequence recognized by the monoclonal antibody with expressed protein, wherein the binding is presented as a relative binding activity relative to the binding to a protein expressed from the gene from MN strain (100%). Horizontal lines indicate mean values for each of the amino acid sequences. In Fig. 3, "1" to "33" are expressed proteins having the following sequences in the middle portion of V3 region: 1: IAPGRAF; 2: IAPGRAL; 3: IAPGSAF; 4: IGLGRAF; 5: IGPARAF; 6: IGPGGAF; 7: IGPGKAF; 8: IGPGRAF; 9: IGPGRAI; 10: IGPGRAL; 11: IGPGRAS; 12: IGPGRAV; 13: IGPGRAW; 14: IGPGRAY; 15: IGPGRPF; 16: IGPGRRF; 17: IGPGRSF; 18: IGPGRSV; 19: IGPGRTF; 20: IGPGRTL; 21: IGPGRTV; 22: IGPGRVF; 23: IGPGRVY; 24: IGPGSAF; 25: IGSGRAF; 26: LGPGGAF; 27: LGPGRAF; 28: MGPGGAF; 29: MGPGKAF; 30: MGPGRAF; 31: MGPGRVY; 32: VGPGRAL; 33: VGPGRAV.

### BEST MODE FOR CARRYING OUT THE INVENTION

In accordance with the method of the present invention as characterized in the appended claims, for assessing fitness of patients for administration of a monoclonal antibody preparation and selecting patients to be administered with said monoclonal antibody preparation with its expected efficacy, it is indispensable to compare an amino acid sequence of a target molecule from patients with a previously determined "reference sequence". The term "reference sequence" as used herein refers to an amino acid sequence of an epitope region of an antigen, which amino acid sequence a monoclonal antibody as an active ingredient of a monoclonal antibody preparation to be administered may recognize and bind to. After such an amino acid sequence of an epitope region is determined as "reference sequence", correlation between efficacy (e.g. neutralizing activity) of said monoclonal antibody and "reference sequence" may be investigated. In case that diversity is seen in an amino acid sequence of an epitope region, e.g. human immunodeficiency virus type 1 (HIV-1), a universal sequence commonly shared among diverse amino acid sequences of said epitope region is determined as "reference sequence".

As such, by previously determining an amino acid sequence of an epitope region recognized and bound by a monoclonal antibody as an active ingredient of a monoclonal antibody preparation, and by determining an amino acid sequence to which said monoclonal antibody may manifest its efficacy as "reference sequence", assessment of fitness of patients for administration of said monoclonal antibody preparation becomes possible through comparison with said "reference sequence".

For the purpose of the present invention, (1) analysis of an amino acid sequence ("reference sequence") of an epitope region recognized by a monoclonal antibody; (2) analysis of a nucleotide sequence that encodes a region encompassing an epitope region of a target molecule present in a biopsy from patients; and (3) acquisition of data concerning correlation between diversity in an amino acid sequence of an antigenic protein and a potency of a monoclonal antibody, need previously be performed.

Specifically, the present invention may be performed in the following processes. For a typical antigen recognized by a monoclonal antibody of interest, binding of said monoclonal antibody to peptide fragments of a protein is investigated to identify an epitope region of said antigen. The peptide fragments may be prepared by digestion of an antigenic protein with a proteolytic enzyme or chemical synthesis of the peptide fragments based on known amino acid sequence information. Analysis of reactivity of said peptide fragments and a monoclonal antibody includes an immunochemical method such as ELISA and dot blot as well as a method using surface plasmon resonance biosensor. Antigens present within the living body include those of exogenous origin such as viruses, bacteria and toxins and of endogenous origin such as cancer specific antigens and disease-related molecules.

For determining a nucleotide sequence of said epitope region in an antigenic protein present within the living body of patients, a region that flanks said epitope region and has scarcely any variance in an amino acid sequence is selected and based on a nucleotide sequence of said region primers for amplification of a nucleic acid are designed. For analysis of a nucleotide sequence of the epitope region of said antigen, using a biological sample such as blood or a tissue, a DNA encoding the epitope region is amplified by PCR using as a template a cDNA obtained from RNAs with a reverse transcriptase or a DNA as well as said primers. The amplified DNAs are sequenced directly or after cloning with a DNA sequencer.

Reactivity of a monoclonal antibody to an antigen having a variety of amino acid sequences in an epitope region may be determined by cloning a DNA obtained as described above containing an epitope region and then measuring binding of said monoclonal antibody to a protein which is expressed in E. coli from the cloned DNA. Furthermore, correlation between an amino acid sequence of said epitope region and efficacy of said monoclonal antibody may be confirmed not only by an antigen-binding activity but also by in vitro, ex vivo or in vivo test using as an index an anti-viral activity or an anti-tumor activity. For example, virus derived from patients is cloned so that an amino acid sequence of said epitope region may be determined from its nucleotide sequence and simultaneously a viral neutralizing activity is measured to thereby provide data of correlation between the amino acid sequence of said epitope region and efficacy of said monoclonal antibody.

A typical example of viruses where diversity in an antigen may be seen is human immunodeficiency virus type 1 (HIV-1). Principal neutralizing domain (PND) of HIV-1 is localized at the middle portion of the 3rd variable region (V3 region) of the envelope glycoprotein gp120. The present inventors have prepared a humanized monoclonal antibody to PND and formulated said antibody for clinical application. Said monoclonal antibody was affected for its neutralizing activity to HIV-1, i.e. binding to an antigen of HIV-1, due to variance in an amino acid sequence of PND region. Thus, for clinical application of said antibody, it was estimated that analysis of an amino acid sequence of the epitope region and subsequent selection of subjects to be administered with said antibody will lead to enhancement of efficacy of said antibody.

As such, in order to first confirm the presence of an antigen to which said monoclonal antibody is capable of binding, correlation between an amino acid sequence of an epitope region and binding of said monoclonal antibody to the epitope region was investigated to determine the amino acid sequence recognized by said monoclonal antibody with its efficacy being expected by the following processes:
(1) Using synthetic peptide fragments consisting of the sequence of PND region, an epitope region sequence recognized by said monoclonal antibody was confirmed.
(2) A V3 region gene was amplified and cloned from a virus isolated from patients and expressed as a fusion protein with β-galactosidase. A binding of said monoclonal antibody to the expressed protein of said V3 region was determined and correlation between the amino acid sequence of the epitope region and binding of said monoclonal antibody was investigated.
(3) Based on the thus obtained results, candidate "reference sequence" of said monoclonal antibody was selected.
(4) A neutralizing activity of said monoclonal antibody to HIV-1 was determined to confirm that correlation exists between the "reference sequence" described above and the neutralizing activity.
(5) HIV-1 V3 region genes were amplified from plasma or peripheral mononuclear cells from patients and their nucleotide sequences were determined. Amino acid sequences were deduced from the obtained nucleotide sequences and compared with the "reference sequence" described above in order to predict fitness of the patients of interest to said monoclonal antibody.

Based on the findings as described above, it was found that diversity of an antigen expressed in patients may be analyzed on the basis of a nucleotide sequence so as to predict binding of said antigen and an antibody to said antigen. For use in clinical scenes, where HIV-1 is the target molecule, a method for selecting subjects to be administered with a monoclonal antibody preparation is provided using plasma from patients as a sample by the processes as described below.

HIV-1 RNAs in plasma are converted to DNAs with a reverse transcriptase and amplification of the nucleic acid of V3 region is performed. The amplified DNA fragments, directly or after cloning, are analyzed for their nucleotide sequences. Amino acid sequences are then deduced from the obtained nucleotide sequences and compared with the previously selected "reference sequence" of the amino acid sequence that is recognizable by the monoclonal antibody in said monoclonal antibody preparation. The "reference sequence" may be determined by measuring binding to peptides or expressed proteins or HIV-1 neutralizing activity of said monoclonal antibody as described above. Besides, by analyzing correlation between the amino acid sequence of PND region of HIV-1 from patients and efficacy of said monoclonal antibody preparation through clinical tests, more particular data of "reference sequence" may be obtained.

The present invention is explained in more detail by means of the following Examples but should not be construed to be limited thereto.

### Example 1

### Analysis of amino acid sequence recognized by anti-HIV monoclonal antibody using synthetic peptides

An amino acid sequence recognized by KD-247, a humanized monoclonal antibody to PND region, was analyzed using synthetic peptides derived from the middle portion of V3 region of HIV-1 gp120. Peptides were synthesized by Pepscan technique (Geysen, H.M. et al., Proc. Natl. Acad. Sci. USA, 81, 3998-4002, (1984)) and binding activity of KD-247 to these peptides was determined by ELISA. In order to determine the shortest amino acid sequence that is recognizable by KD-247, a peptide of the ten amino acid residues "IHIGPGRAFY", which is the amino acid sequence of the middle portion of V3 region of laboratory HIV-1 strain (MN strain) that is neutralized by KD-247, and peptides wherein amino acid residues were sequentially deleted one by one from the above peptide "IHIGPGRAFY" to the extent of peptides with four amino acid residues in length were synthesized as follows: "IHIGPGRAF", "HIGPGRAFY", "IHIGPGRA", "HIGPGRAF", "IGPGRAFY", "IHIGPGR", "HIGPGRA", "IGPGRAF", "GPGRAFY", "IHIGPG", "HIGPGR", "IGPGRA", "GPGRAF", "PGRAFY", "IHIGP", "HIGPG", "IGPGR", "GPGRA", "PGRAF", "GRAFY", "IHIG", "HIGP", "IGPG", "GPGR", "PGRA", "GRAF", and "RAFY".

Polyethylene rods bound with the synthetic peptides were precoated with a phosphate buffered saline containing 2% bovine serum albumin and 0.1% Tween 20 and reacted with 2 µg/ml of KD-247. KD-247 bound with the peptides was measured with peroxidase-labeled anti-human κ antibody and the substrate. As shown in Fig. 1, the shortest amino acid sequence was found to be "IGPGR".

In order to investigate if reactivity of KD-247 may be altered when each of the amino acid residues of the peptide recognized by KD-247 is replaced with other amino acid residues, peptides wherein each one of the amino acid residues in the sequence "IHIGPGRAFY" derived from HIV-1 (MN strain) was replaced with the other 19 naturally occurring amino acid residues were synthesized and reactivity of these peptides with KD-247 was measured as described above. As shown in Fig. 2, it was confirmed that only few other amino acid residues may be replaced for the middle portion sequence "PGR", especially the arginine (R) residue is an essential amino acid residue. For the amino acid residues other than "PGR", they could be replaced with many other amino acid residues.

From the results as described above, it was found that KD-247 basically recognized "IGPGR" sequence and could still recognize those amino acid sequences with replacement of an amino acid residue within "IGPGR" sequence and/or its vicinity.

### Example 2

### Analysis of amino acid sequence recognized by anti-HIV monoclonal antibody using expressed proteins

The recognition site of KD-247 as confirmed in Example 1 was based on the observation of the reaction between the short peptides and said antibody. For assessing an antibody binding to an antigenic protein, however, steric influence needs be considered in addition to the amino acid sequence of the binding portion of the antigen. Thus, binding of KD-247 to an expressed protein containing V3 region was determined.

A gene containing HIV-1 V3 region was first amplified by nested PCR technique using as a template cDNAs obtained from HIV-1 RNA genes from plasma of patients infected with HIV-1 with a reverse transcriptase or HIV-1 proviral DNAs of peripheral blood mononuclear cells. Primers used for the first PCR were: 5'-ACACATGGAATTAGGCCAGT-3' (OA-4) (SEQ ID NO: 1) and 5'-AAATTCCCCTCCACAATTAA-3' (OD-4) (SEQ ID NO: 2), and primers used for the second PCR were: 5'-GCCGGATCCTCAACTCAACTGCTGTTAAAT-3' (EB-2) (SEQ ID NO: 3) and 5'-GCTCTGCAGTCAAATTTCTGGGTCCCCTCCTGAGG-3' (EC-2) (SEQ ID NO: 4). The amplified DNA fragments were purified, cleaved with restriction enzymes BamHI and PstI and cloned into vector plasmids (pUEXI) containing a β-galactosidase (β-Gal) gene. The resulting vectors were introduced into competent cells for cloning. PCR was performed using the cloned DNAs as a template and EB-2 and EC-2 primers and a nucleotide sequence of V3 region was analyzed using the amplified DNAs as a template and a DNA sequencer. Based on the obtained nucleotide sequence, an amino acid sequence of V3 region was determined.

On the other hand; each of the cloned E. coli cells where the genetic sequence was analyzed were cultured and a fusion protein of V3 region and a beta-galactosidase (V3/β-Gal) was obtained as described below. First, the cultured E. coli cells were disrupted with a cell-disrupting device, the disrupted cells were centrifuged, and the precipitates were dissolved in Tris buffer (pH7.5) containing 0.5% Triton X-100 followed by further centrifugation. The precipitates containing inclusion bodies were dissolved in Tris buffer (pH 7.5) containing 8M urea to purify V3/β-Gals. The obtained V3/β-Gals were subjected to SDS-PAGE so as to confirm that the fusion proteins were expressed with no abnormality. Then, ELISA was performed in order to adjust a concentration of V3/β-Gals. Using a plate with an immobilized anti-β-Gal antibody, either the expressed V3/β-Gals or commercially available β-Gal was added thereto. Peroxidase-labeled anti-β-Gal antibody was used as a detector antibody. A calibration curve was prepared with the results obtained from the standard and a concentration of the expressed V3/β-Gals was determined in terms of a concentration of β-Gal.

Then, in order to assess reactivity between V3/β-Gals and KD-247, additional ELISA was performed. Plates were immobilized with the anti-β-Gal antibody and were reacted with 200 ng/ml of each of V3/β-Gals. To the captured V3/β-Gals was reacted with 1 µg/ml of KD-247 and the reaction was detected with a peroxidase-labeled anti-human IgG antibody. Binding of KD-247 to each of V3/β-Gals was presented relative to the absorbance (100%) of a positive control as set in each of the plates, i.e. V3/β-Gal from HIV-1 MN strain.

Fig. 3 shows a relative binding activity of KD-247 to the amino acid sequences of the V3 middle portion of each of V3/β-Gals wherein about 120 sorts of HIV-1 clones from patients were analyzed. The clones that exhibited 100% or more of the relative binding had the amino acid sequences of the middle portion of V3 region: "IGPARAF" (SEQ ID NO: 5), "IGPGRSF" (SEQ ID NO: 6), "IGPGRAL" (SEQ ID NO: 7), "IGPGRTF" (SEQ ID NO: 8), "IGPGRAI" (SEQ ID NO: 9), "VGPGRAL" (SEQ ID NO: 10), and "IGPGRAF" (SEQ ID NO: 11). These sequences may be referred to as exemplary "reference sequences" for assessing fitness to KD-247.

### Example 3

### Analysis of amino acid sequence of HIV-1 V3 region and viral neutralizing activity

Correlation between the amino acid sequences of V3 region and the neutralizing activity was determined using laboratory strains and clinically isolated strains of HIV-1.

Nucleotide sequences of HIV-1 V3 region were determined as described in Example 2. The viruses were reacted with varied concentrations of KD-247 at 37°C for 1 hour and then inoculated into peripheral blood mononuclear cells from healthy adults activated with phytohemagglutinin. After culture for 7 days, the cells were washed and a culture was further continued in the presence of IL-2 for 7 days. An amount of HIV-1 p24 antigen in the culture supernatant was measured by ELISA. The amount of p24 antigen obtained in the absence of the antibody was taken as 100% and each concentration of KD-247 added that reduced 50% (IC₅₀) or 90% (IC₉₀) of p24 antigen were presented as the neutralizing activity.

As shown in Table 1, the neutralizing activity of KD-247 was dependent upon the amino acid sequences in the middle portion of V3 region. Namely, all the HIV-1 viruses that had the "IGPGRAF" sequence in the middle portion of V3 region, binding of which sequence to KD-247 was confirmed in Examples 1 and 2, were neutralized by KD-247. This indicates that the amino acid sequences in the middle portion of V3 region have correlation with the binding activity and the neutralizing activity of KD-247 to said virus.

**Table 1**

| HIV-1 Strain | Amino acid sequence of V3 region | | Neutralizing activity (*µ*g/ml) | |
|---|---|---|---|---|
| | | | IC₉₀ | IC₅₀ |
| Laboratory strain | | | | |
| MN | CTRPNYNKRKRIHI | GPGRAFYTTKNIIGTIRQAHC | 1 | 0.1 |
| IIIB | -----N-T---KS-QR | ------V-IGK- -NM----- | >50 | >50 |
| SF2 | -----N-T--G--- | -------A-EK-V-D------ | 5 | 1 |
| AD8 | -----N-T--S--- | ---------GD---D------ | 10 | 5 |
| 89.6 | -----N-T-R-LS- | -------ARR----D------ | 2.5 | 0.2 |
| Clinically isolated strain | | | | |
| 1 | ----- N-R-T---- | --------------------- | 5 | 0.3 |
| 2 | ----SN-K--S--- | ----------GE---D------ | 5 | 0.4 |
| 3 | -----N-T--S--- | ---------GE---D------ | 8 | 0.6 |
| 4 | -----N-7--S--M | ---K-----GD---N----Y- | >50 | >50 |
| 5 | ---I-N-T--S--- | -------A-GE---N-K---- | 10 | 1.4 |
| 6 | -----N-T--S-R-QR | ------V-IGK -MM----- | >50 | >50 |
| 7 | -I---N-T--G--- | -L-WK-A-G--H----- | >50 | >50 |
| 8 | -----N-T--S-R-QR | ------V-IGK--NM----- | >50 | >50 |
| 9 | --G--N-T--S-R-QR | ------V-IGK-NM----- | >50 | >50 |
| 10 | -I---N-T--G--- | -------A-D---N------- | 8 | 1.2 |
| 11 | ----HKTI------ | ----------Q-E-N----- | 5 | 0.4 |
| 12 | ----SN-T-R---- | ---------RQ-R-D----- | 4 | 0.2 |
| 13 | -----N-1--H--- | ---------RG-RD--K--- | 10 | 0.6 |
| 14 | -------T--G--- | ------V---G-RD--K--- | 4 | 0.3 |
| 15 | -----N-T--S-L- | ---Q-W---GQ---D------ | >50 | >50 |
| 16 | -----N-T--S-PL | ---Q-W---GQ-L-D------ | >50 | >50 |
| 17 | ----SN-T-TS-T- | ---QV--R-GD---D------ | >50 | >50 |
| 18 | ----SN-T-TS-T- | ---QV--R-GD---D------ | >50 | >50 |

| | | | | |
|---|---|---|---|---|
| - :The same amino acid residue as that MN stain | | | | |

### Example 4

### Analysis of amino acid sequence of V3 region in clinical sample and test for fitness of patients to KD-247

A HIV-1 gene was analyzed from plasma or peripheral blood mononuclear cells from patients as described in Example 2. Based on this analysis, an amino acid sequence of HIV-1 V3 region was deduced and compared with "reference sequence", i.e. the amino acid sequences that were confirmed to be strongly reactive with KD-247 in Example 2, to test fitness of each of patients to KD-247.

Table 2 shows amino acid sequences deduced from the HIV-1 gene in plasma or peripheral blood mononuclear cells from patients and fitness of each of patients to KD-247. The amino acid sequences enclosed by the dotted line were compared with the "reference sequences" (SEQ ID NO: 5 to SEQ ID NO: 11) obtained in Example 2. When the amino acid of interest was consistent with one of the "reference sequences", it was assessed as being "fit" whereas when the amino acid of interest was not consistent with any one of the "reference sequences", it was assessed as being "unfit".

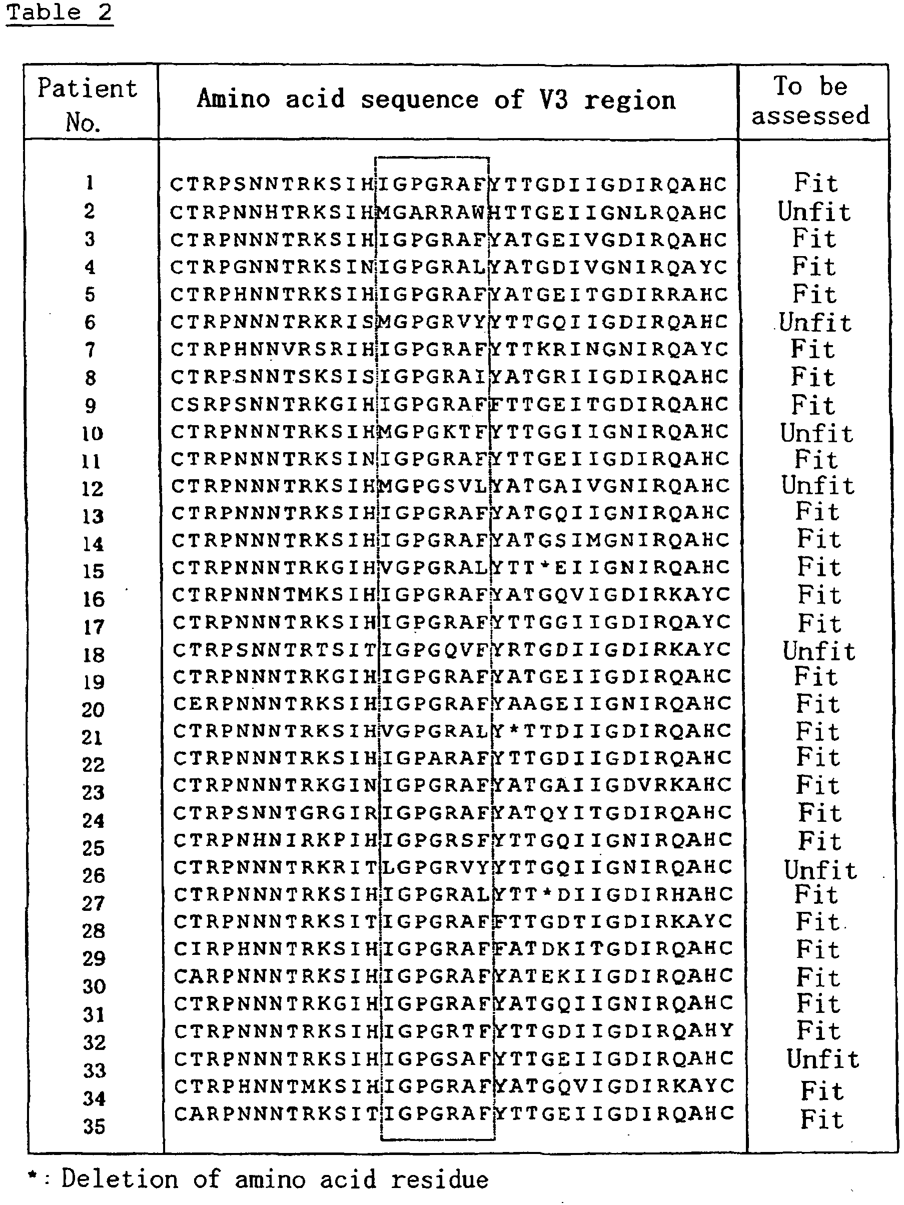

## Claims

1. A method for enhancing efficacy of a monoclonal antibody preparation, wherein said monoclonal antibodyis an antibody that recognizes as an antigen human immunodeficiency virus type 1 (HIV-1), which comprises selecting patients fitted for administration of said monoclonal antibody preparation by previously analyzing an amino acid sequence of an antigenic protein of a target molecule present in a sample from patients, wherein said method comprises steps: (1) deducing an amino acid sequence of a protein expressed in patients from a nucleotide sequence of a gene of a target molecule determined by isolation and analysis of said gene in biopsy from patients; (2) assessing fitness of patients for administration of said monoclonal antibody preparation by comparing the amino acid sequence deduced in step (1) with an amino acid sequence that is recognizable by said monoclonal antibody as previously determined; and (3) selecting patients to be administered with said monoclonal antibody preparation with its expected efficacy based on the fitness assessed in step (2).

2. The method for enhancing efficacy of a monoclonal antibody preparation of claim 1, wherein comparison between a deduced amino acid sequence of a target protein and an amino acid sequence(s) that is/are recognizable by said monoclonal antibody as previously determined is carried out using as an index binding of a monoclonal antibody to a peptide or an expressed protein having said deduced amino acid sequence.

3. The method for enhancing efficacy of a monoclonal antibody preparation of claim 1 or 2, wherein said monoclonal antibody is an antibody to V3 region of envelop glycoprotein gp120 of human immunodeficiency virus type 1 (H1V-1).

4. The method for enhancing efficacy of a monoclonal antibody preparation of any one of claims 1 to 3, wherein the amino acid sequence(s) that is/are recognizable by said monoclonal antibody as previously determined for HIV-1 is selected from the group consisting of "IGPARAF" (SEQ ID NO: 5), "IGPGRSF" (SEQ ID NO: 6), "IGPGRAL" (SEQ ID NO: 7), "IGPGRTF" (SEQ ID NO: 8), "IGPGRAI" (SEQ ID NO: 9), "VGPGRAL" (SEQ ID NO: 10), and "IGPGRAF" (SEQ ID NO: 11).

## Patentansprüche

1. Verfahren zur Erhöhung der Wirksamkeit eines monoklonalen Antikörperpräparats, wobei der monoklonale Antikörper ein Antikörper ist, der als Antigen das humane Immundefizienz-Virus Typ 1 (HIV-1) erkennt, welches das Auswählen von für die Verabreichung des monoklonalen Antikörperpräparats geeigneten Patienten umfasst, wobei vorher die Aminosäuresequenz eines in einer Probe von Patienten vorhandenen antigenen Proteins des Zielmoleküls analysiert wird, wobei das Verfahren die Schritte umfasst: (1) das Ableiten einer Aminosäuresequenz eines in Patienten exprimierten Proteins von einer Nukleinsäuresequenz eines Gens eines Zielmoleküls, die durch Isolation und Analyse des Gens in einer Biopsie von Patienten bestimmt wird; (2) das Bestimmen der Eignung von Patienten für die Verabreichung des monoklonalen Antikörperpräparats durch Vergleich der in Schritt (1) abgeleiteten Aminosäuresequenz mit einer Aminosäuresequenz, die gemäß vorheriger Ermittlung vom monoklonalen Antikörper erkannt wird; und (3) das Auswählen von Patienten, denen das monoklonale Antikörperpräparat mit der erwarteten Wirksamkeit verabreicht werden soll, basierend auf der in Schritt (2) festgestellten Eignung.

2. Verfahren zur Erhöhung der Wirksamkeit eines monoklonalen Antikörperpräparats nach Anspruch 1, wobei der Vergleich zwischen einer abgeleiteten Aminosäuresequenz eines Zielproteins und (einer) Aminosäuresequenz(en), die gemäß vorheriger Ermittlung vom monoklonalen Antikörper erkannt wird/werden, durch die Verwendung der Bindung eines monoklonalen Antikörpers an ein Peptid oder ein exprimiertes Protein, das die abgeleitete Aminosäuresequenz besitzt, als Index ausgeführt wird.

3. Verfahren zur Erhöhung der Wirksamkeit eines monoklonalen Antikörperpräparats nach Anspruch 1 oder 2, wobei der monoklonale Antikörper ein gegen die V3-Region des Hüllglykoproteins gp120 des humanen Immundefizienz-Viruses Typ 1 (HIV-1) gerichteter Antikörper ist.

4. Verfahren zur Erhöhung der Wirksamkeit eines monoklonalen Antikörperpräparats nach einem der Ansprüche 1 bis 3, wobei die Aminosäuresequenz(en), die gemäß vorheriger Ermittlung für HIV-1 vom monoklonalen Antikörper erkannt wird/werden, "IGPARAF" (SEQ ID Nr:5), "IGPGRSF" (SEQ ID Nr:6), "IGPGRAL" (SEQ ID Nr:7), "IGPGRTF" (SEQ ID Nr:8), "IGPGRAI" (SEQ ID Nr:9), "VGPGRAL" (SEQ ID Nr:10), oder "IGPGRAF" (SEQ ID Nr:11) ist/sind.

## Revendications

1. Méthode pour augmenter l'efficacité d'une préparation d'anticorps monoclonal, dans laquelle ledit anticorps monoclonal est un anticorps qui reconnait en tant qu'antigène le virus de l'immunodéficience humaine de type 1 (VIH-1), qui comprend la sélection de patients appropriés pour une administration de ladite préparation d'anticorps monoclonal en analysant préalablement une séquence d'acides aminés d'une protéine antigénique d'une molécule cible présente dans un échantillon provenant des patients, dans laquelle ladite méthode comprend les étapes de : (1) déduction d'une séquence d'acides aminés d'une protéine exprimée chez des patients à partir d'une séquence nucléotidique d'un gène d'une molécule cible déterminé par isolement et analyse dudit gène dans une biopsie des patients ; (2) évaluation de l'aptitude des patients pour l'administration de ladite préparation d'anticorps monoclonal en comparant la séquence d'acides aminés déduite à l'étape (1) avec une séquence d'acides aminés qui est reconnaissable par ledit anticorps monoclonal tel que précédemment déterminé ; et (3) sélection des patients devant recevoir ladite préparation d'anticorps monoclonal avec son efficacité attendue basée sur l'aptitude évaluée à l'étape (2).

2. Méthode pour augmenter l'efficacité d'une préparation d'anticorps monoclonal selon la revendication 1, dans laquelle la comparaison entre une séquence d'acides aminés déduite d'une protéine cible et une ou des séquence(s) d'acides aminés qui est/sont reconnaissable(s) par ledit anticorps monoclonal tel que précédemment déterminé est réalisée en utilisant en tant qu'index la liaison d'un anticorps monoclonal à un peptide ou à une protéine exprimée ayant ladite séquence d'acides aminés déduite.

3. Méthode pour augmenter l'efficacité d'une préparation d'anticorps monoclonal selon la revendication 1 ou 2, dans laquelle ledit anticorps monoclonal est un anticorps de la région V3 d'une glycoprotéine d'enveloppe gp120 du virus de l'immunodéficience humaine de type 1 (VIH-1).

4. Méthode pour augmenter l'efficacité d'une préparation d'anticorps monoclonal selon l'une quelconque des revendications 1 à 3, dans laquelle la/les séquence(s) d'acides aminés qui est/sont reconnaissables par ledit anticorps monoclonal tel que précédemment déterminé pour le VIH-1 est choisi dans le groupe consistant en « IGPARAF » (SEQ ID NO : 5), « IGPGRSF » (SEQ ID NO : 6), « IGPGRAL » (SEQ ID NO : 7), « IGPGRTF » (SEQ ID NO : 8), « IGPGRAI » (SEQ ID NO : 9), « VGPGRAL » (SEQ ID NO : 10), et « IGPGRAF » (SEQ ID NO : 11).
